# EUROPEAN PATENT APPLICATION

(11) **EP 0 700 910 A1**
(43) Date of publication of application: **13.03.1996**
(21) Application number: 95113913.8
(22) Date of filing: 05.09.1995
(51) Int. Cl.: C07D 305/14, C07B 63/00

(54) **Process for isolation of taxol from Taxus sumatrana**

(30) Priority: 06.09.1994 JP 239444/94
(71) Applicant: SHIONOGI & CO., LTD., Osaka-shi, Osaka-fu 541 (JP)
(72) Inventor: Kitagawa, Isao, Toyonaka-shi, Osaka 560 (JP); Shibuya, Hirotaka, Fukuyama-shi, Hiroshima 720 (JP); Roemantyo, Bogor, Jawa Barat (ID); Kobayashi, Motomasa, Ao-gein Minoo-shi, Osaka 562 (JP)
(74) Representative: VOSSIUS & PARTNER

(57) **Abstract**

This invention relates to a process for preparing taxol characterized by that taxol is extracted from Taxus sumatrana (Taxaceae) and purified. According to this invention, taxol can be obtained efficiently.

## Description

This invention relates to a process for isolating taxol represented by the formula (I) from a plant material. 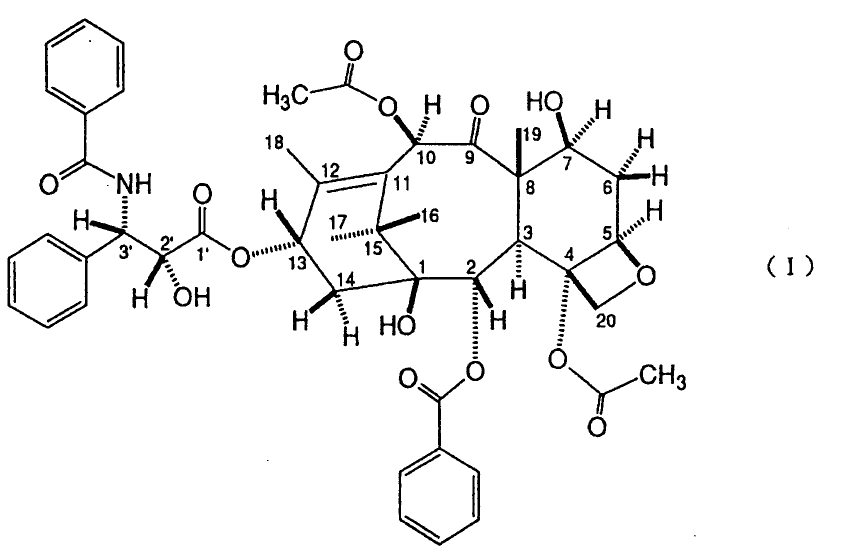
Taxol shows marked efficacy against malignant tumors such as ovarian cancer, breast cancer, etc. and is of great value as a medicament.

Taxol is a known compound having antitumor activity. In 1963, researchers at National Cancer Institute of USA (NCI) discovered that a potent antitumor substance occurs in an extract of the bark of Taxus brevifolia, a yew tree, the North America of origin. They subsequently isolated the active substance, identified it to be a compound of the above formula, and named it taxol (M.C. Wani, et al., J. Am. Chem. Soc. 93: 2325, 1971).

It was subsequently elucidated that taxol has potent antitumor activity against a variety of malignant tumors such as cancers of the ovary, uterus and mammary glands and that it acts on tumor cells which are not responsive to cisplatin. Today, taxol has attracted the attention as a very promising anticancer agent.

Taxol can be isolated from the bark of North American yew trees as mentioned above. However, this compound occurs in very small amounts in the barks and the growth of the trees is extremely slow, so that it could not be procured in sufficient quantities. Moreover, use of the standing tree bark is subject to many restrictions from the standpoint of protection of forests.

Because not only taxol but also its related substances (taxoids) are isolated from North American yew trees as mentioned above, a research has been carried out on the chemical conversion of such related substances into taxol (International Patent Publication WO93/16059). Furthermore, chemical synthesis of taxol has also been investigated but because of its extremely complex chemical structure, a process for total synthesis remains to be established yet US-A-5274137).

Efficient mass production of taxol has been a very important subject of endeavor for furtherance of clinical studies on taxol and relief of cancer patients.

Under these circumstances, this invention has for its object to provide a process for efficient isolation of taxol.

The gist of this invention resides in that, to obtain taxol, taxol is isolated by extracting from Taxus sumatrana (Taxaceae). In the preferred embodiment, this invention is the process for isolating taxol by extraction of the leaves of said Taxus sumatrana.

Said Taxus sumatrana according to this invention is a plant of the genus Taxus which is native to Sumatra, Indonesia. Its leaves can be harvested on a steady basis without causing death of the tree to benefit the protection of nature.

According to this invention, a large amount of taxol can be obtained efficiently from Taxus sumatrana.

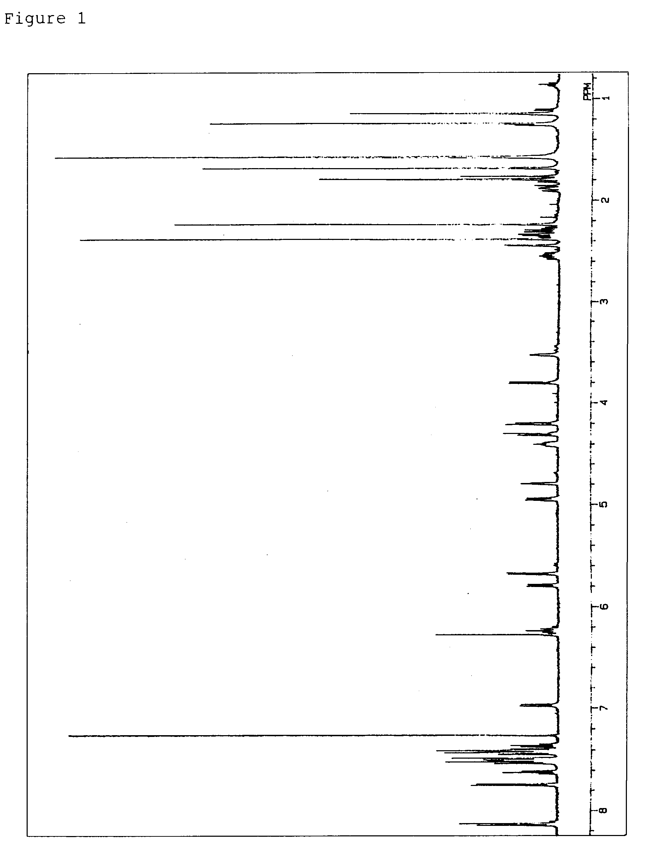

Figure 1 shows the ¹H-NMR spectrum of taxol obtained in Example 1.

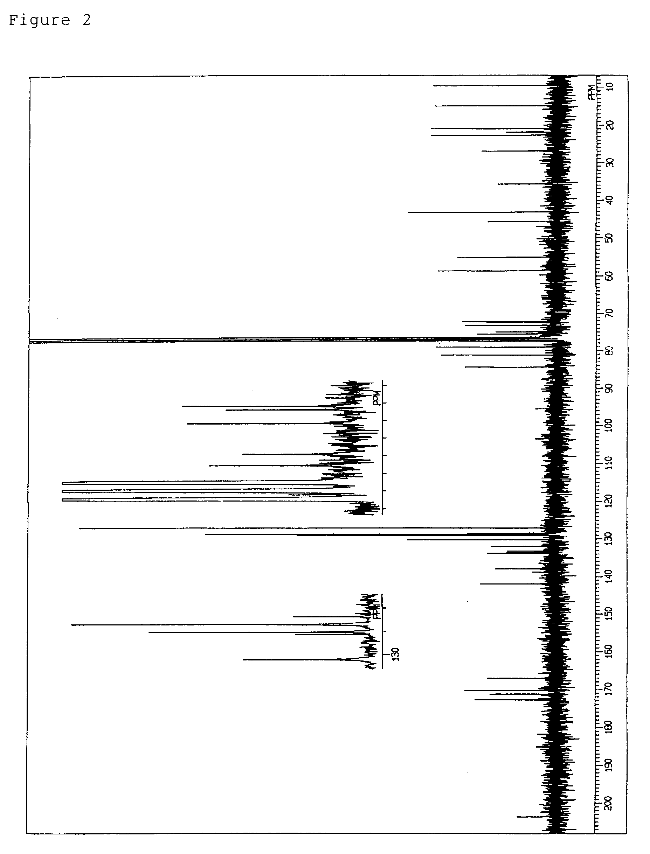

Figure 2 shows the ¹³C-NMR spectrum of taxol obtained in Example 1.

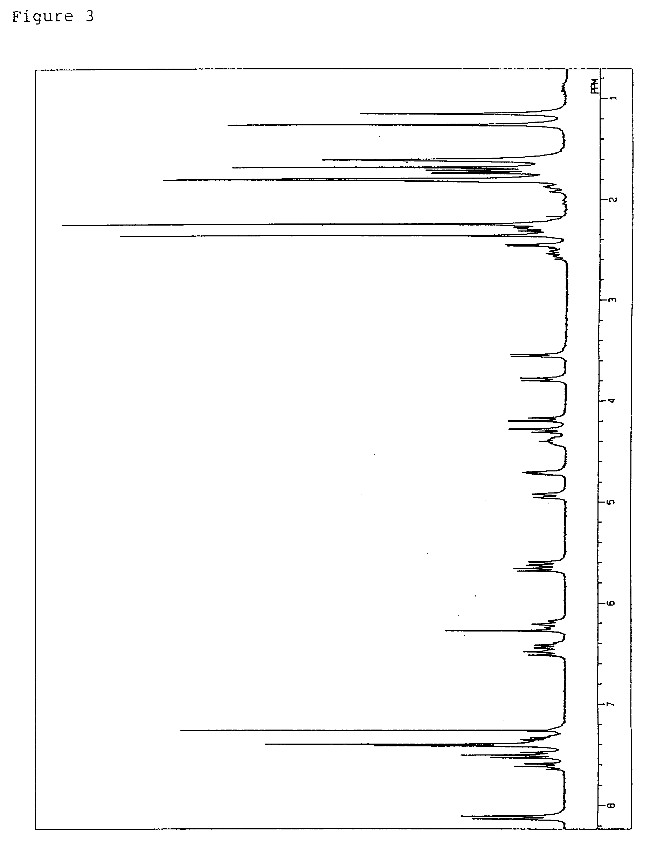

Figure 3 shows the ¹H-NMR spectrum of cepharomannine obtained in Example 1.

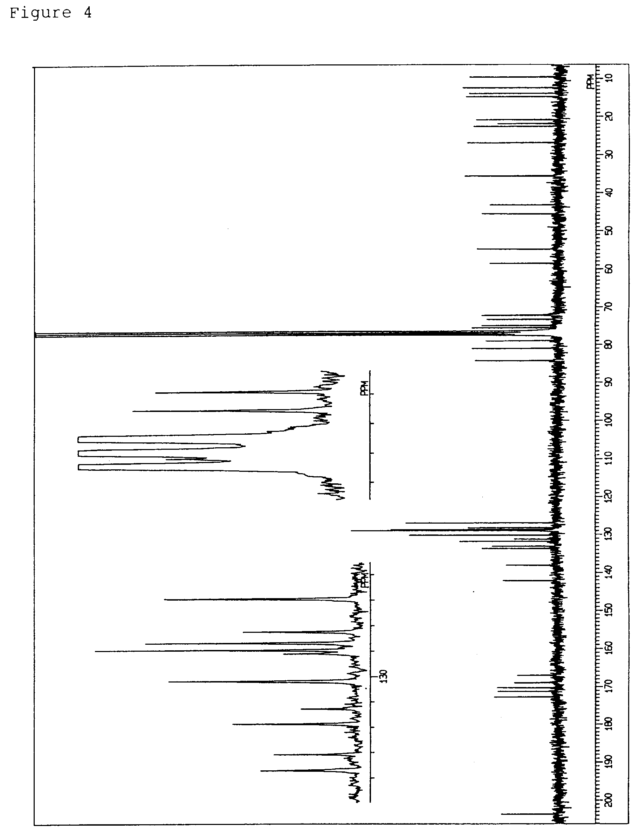

Figure 4 shows the ¹³C-NMR spectrum of cepharomannine obtained in Example 1.

The process for isolation according to this invention comprises a step of extraction where a starting material is the leaves of said Taxus sumatrana and a step of purification of the extract resulting from said extraction steps.

In said extraction step, the leaves of Taxus sumatrana may be raw but preferably, the dried ones may be used to increase the efficiency of extraction, and finely cutleaves are much better.

In said extraction step, there is no restriction, i.e. in preparing the extract from Taxus sumatrana leaves, an organic solvent may be utilized or not be used. However, because taxol is insoluble in water, the use of an organic solvent is preferred.

There is no limitation on the kind of organic solvent for extraction and virtually any of the common organic solvents can be employed. The preferred organic solvent includes but is not limited to alcohols such as methanol, ethanol, etc., dichloromethane, benzene, chloroform, dioxane and acetone. Particularly, methanol and dichloromethane are preferred. These organic solvents can be used singly or in combination.

In extracting, the leaves of Taxus sumatrana are mixed with a solvent and allowed to stand under cooling or heating, preferably at room temperature or under heating, for a period of several hours to several days. Said extraction procedure may be carried out under acidic - alkaline conditions, but it is preferably conducted under neutral conditions in order to prevent degradation of the objective compound. Said extraction can be carried out by any known procedure for extracting pharmacologically active principles from plant sources. In the preferred extraction procedures, the leaves are soaked in a chilled organic solvent or extracted with an organic solvent under reflux of the solvent used. The supercritical fluid extraction process using a mixture of carbon dioxide and ethanol (The Journal of Supercritical Fluids, 5, 1, 1992) can also be employed. These procedures can be used in combination as a multi-stage extraction process.

From the extract obtained by the above extraction step, the solvent may be removed by distillation, if desired, by a suitable means to provide an extract concentrate. Such distillation can be carried out in a conventional manner, for example, under reduced pressure or heating.

Then, the extract is subjected to the purification step as mentioned above. Thus, the extract is first partitioned between a water-immiscible organic solvent and water and the organic fraction is recovered. The partition system between a water-immiscible organic solvent and water is not particularly restricted. Said partition may be carried out in the conventional manner, for example, using ethyl acetate-water, methylene chloride-water, n-butanol-water, or benzene-water and the like. The preferred system is ethyl acetate-water.

Then, the organic solvent fraction recovered as above is concentrated by a suitable procedure if necessary, and purified by chromatography.

This concentration can be carried out by a conventional technique, for example, by the same distillation procedure as described above. Said chromatography may also be any of the known methods, for example, column or thin-layer chromatography. The column chromatography is not especially restricted, and a conventional method can be employed. For example, adsorption chromatography using silica gel or the like or gel permeation chromatography using Sephadex® or the like can be used. Supercritical fluid chromatography or high performance liquid chromatography (HPLC) can also be employed. These chromatographic procedures can be used singly or through multiple purification stages.

In the practice of this invention, not only taxol but also certain structural analogs of taxol such as cephalomannine, represented by formula (II) are obtained in some cases. 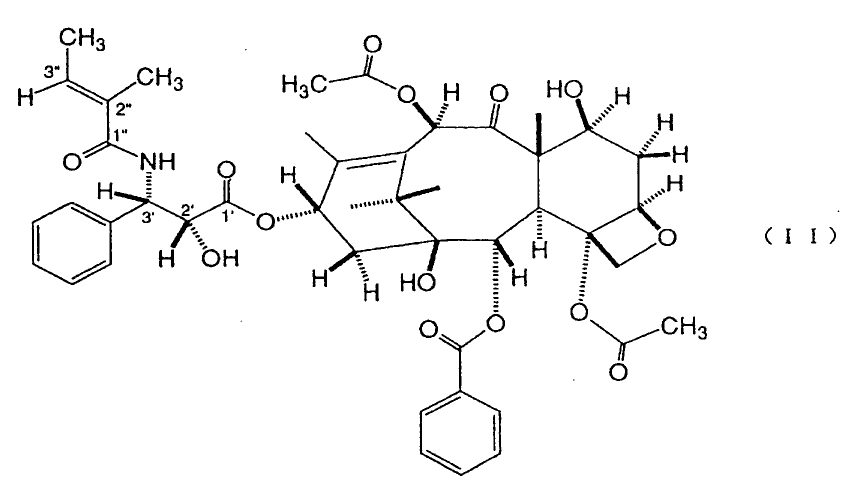
When a mixture of taxol and its related compounds is obtained, the latter compounds can be subsequently oxidized with, for example, OsO₄ to obtain taxol in a pure form. If desired, the mixture may be applied to separation and purification by HPLC, for instance, to isolate taxol.

Said cephalomannine (II) can be utilized as a intermediate for synthesis of taxol and per se is expected to have useful pharmacological activities.

Taxus sumatrana (Taxaceae) according to this invention was not known as an extraction source of taxol but was established for the first time by the inventors of this invention to contain taxol and its related compounds. Since the leaf of Taxus sumatrana contains comparatively large amounts of taxol and analogs thereof, it is not necessary to harvest the bark or core wood. Moreover, it is possible to cultivate seedlings and harvest the leaf or subject the leaf tissue to tissue culture.

The following examples further illustrate this invention in details but by no means limit the scope of this invention.

### Example 1

### 1. Extraction of taxol

Fifty grams of dried leaves of Taxus sumatrana were slit into thin slices and then extracted by cold soaking in 500 ml of dichloromethane-methanol(1:1) (2 hours × 3 times). The extracted residue was further extracted with 500 ml of methanol under reflux (3 hours × 2 times). The extracts thus obtained were combined and the solvent was distilled off under reduced pressure to provide 5.14 g of extract. (yield based on dried leaves: 10.28 %)

### 2. Separation and purification of the extract

The extract (5.10 g) was separated by ethyl acetate-water (1:1, 1.2 L) partition and the ethyl acetate fraction was concentrated under reduced pressure to recover 2.43 g of an ethyl acetate soluble fraction (yield based on dried leaves: 4.86 %).

The ethyl acetate soluble fraction (2.40 g) was purified by silica gel column chromatography [silica gel 60 (Merck & Co.) 120 g, eluted with mixed solvent of n-heane: dichrolomethane:ethanol 7:5:1 and, then, with methanol] to obtain 7 fractions [fraction A (415 mg), fraction B (534 mg), fraction C (64 mg), fraction D (285 mg), fraction E (183 mg), fraction F (172 mg) and fraction G (744 mg)]. Fraction D (285 mg) showing the same Rf value as taxol on a thin-layer chromatogram (TLC) was further fractionated by Sephadex LH-20 column chromatography [Sephadex LH-20 (Pharmacia) 400 g, eluted with mixed solvent of dichloromethane:methanol 1:2] into 3 fractions [fraction D1 (46 mg), fraction D2 (189 mg), fraction D3 (44mg)]. Fraction D2 (189 mg) was further fractionated by silica gel column chromatography [SIL-120-S50 (YMC) 15 g, eluted with mixed solvent of n-hexane: dichloromethane:ethanol 8:5:1, and, then, with methanol] into 4 fractions [fraction D21 (61 mg), fraction D22 (19 mg), fraction D23 (68 mg) and fraction D24 (31 mg)]. Fraction D23 (68 mg), which contained taxol, was purified by normal phase HPLC [YMC-Pack SIL, 250 mm× 10 mm ID, eluted with mixed solvent of n-hexsane:dicholromethane:isopropanol 17:5:1] to recover 6 mg of a mixture of taxol (I) and cephalomannine (II). This mixture (6 mg) was purified by reversed phase HPLC (CASPCELL PAK ODS C₁₈SG-120, 250 mm× 10 mm ID, eluted with mixed solvent of methanol:water 67:33) to give 3.0 mg of fraction (I) (yield based on dried leaves: 0.006 %) and 2.5 mg of fraction (II) (yield based on dried leaves: 0.005 %) as white amorphous solids. Based on the following measured physical data, fraction (I) and fraction (II) were identified as taxol (I) and cephalomannine (II), respectively.

The physical data on the fraction (I) obtained above (taxol (I)) are given below.
[α ]_{D} =-43° (c=0.41,MeOH,24°C) lit. [α ]²⁰_{D} =-49° (MeOH) (J. Am. Chem. Soc., 93, 2325, 1971).
FAB-MS(m/z):854(M+H)⁺
IR ν ^{KBr} ₘₐₓ cm⁻¹:3433,2930,1722,1653,1602,1244
UV λ ₘₐₓ (MeOH) nm( ε ):227(25500),265(1800)
¹H-NMR(500MHz,CDCl₃ ,δ ):
5.67(1H,d,J=6.8Hz,2-H)
3.80(1H,d,J=6.8Hz,3-H)
4.94(1H,d,J=7.7Hz,5-H)
2.55(1H,m,6α -H)
1.88(1H,m,6β -H)
4.40(1H,m,7-H)
6.27(1H,s,10-H)
6.23(1H,t,J=8.6Hz,13-H)
2.35(1H,m,14α -H)
2.29(1H,m,14β -H)
1.14(3H,s,16-Me)
1.24(3H,s,17-Me)
1.79(3H,s,18-Me)
1.68(3H,s,19-Me)
4.30(1H,d,J=8.5Hz,20α -H)
4.20(1H,d,J=8.5Hz,20β -H)
4.79(1H,s br,2'-H)
5.79(1H,dd,J=8.5,2.6Hz,3'-H)
6.97(1H,d,J=8.5Hz,3'-NH)
8.13(2H,dd,J=8.6,1.7Hz,2-OBz(ortho)-H)
7.51(2H,m,2-OBz(meta)-H)
7.61(1H,dd,J=8.6,1.7Hz,2-OBz(para)-H)
7.48(2H,m,3'-Ph(ortho)-H)
7.42(2H,m,3'-Ph(meta)-H)
7.35(1H,m,3'-Ph(para)-H)
7.74(2H,dd,J=8.6,1.7Hz,NBz(ortho)-H)
7.40(2H,m,NBz(meta)-H)
7.49(1H,m,NBz(para)-H)
1.76(1H,s,1-OH)
2.45(1H,d,J=4.3Hz,7-OH)
3.53(1H,d,J=5.1Hz,2'-OH)
2.38(3H,s,4-OAc)
2.24(3H,s,10-OAc)
¹³C-NMR(67.8MHz,CDCl₃ ,δ c):
C-1 79.0(s) C-1' 172.7(s)
C-2 74.9(d) C-2' 73.1(d)
C-3 45.6(d) C-3' 55.0(d)
C-4 81.1(s) 4-OCOCH₃ 170.3(s)
C-5 84.4(d) 4-OCOCH₃ 22.6(q)
C-6 35.6(t) 10-OCOCH₃ 171.2(s)
C-7 72.2(d) 10-OCOCH₃ 20.8(q)
C-8 58.6(s) 2-COC₆H₅ 167.0(s)
C-9 203.6(s) 2-COC₆H₅ (q)129.1(s)
C-10 75.5(d) (o)130.2(d)
C-11 133.2(s) (m)128.7(d)
C-12 141.9(s) (p)133.7(d)
C-13 72.4(d) 3'-C₆H₅ (q)133.6(s)
C-14 35.6(t) (o)127.0(d)
C-15 43.1(s) (m)128.7(d)
C-16 21.8(q) (p)131.9(d)
C-17 26.8(q) N-COC₆H₅ 166.9(s)
C-18 14.8(q) N-COC₆H₅ (q)137.9(s)
C-19 9.5(q) (o)127.0(d)
C-20 76.5(t) (m)129.0(d)
(p)128.4(d)
The physical data of the resulting fraction (II) (cephalomannine (II)) was as follows.
[α ]_{D} =-49° (c=0.37,MeOH,24°C), lit. [α ]²⁰_{D} -41° (MeOH)(J.Org.Chem.,46, 1469 (1981))
FAB-MS(m/z):832(M+H)⁺
IRν ^{KBr} ₘₐₓ cm⁻¹:3433,2947,1722,1664,1633,1242
UV λ ₘₐₓ (MeOH) nm( ε ):227sh(25500),265(1800)
¹H-NMR (270MHz,CDCl₃,δ ):
5.67(1H,d,J=7.0Hz,2-H)
3.78(1H,d,J=7.0Hz,3-H)
4.94(1H,d,J=7.6Hz,5-H)
2.55(1H,m,6α -H)
1.88(1H,m,6β -H)
4.40(1H,m,7-H)
6.27(1H,s,10-H)
6.20(1H,t,J=8.6Hz,13-H)
2.35(1H,m,14α -H)
2.29(1H,m,14β -H)
1.15(3H,s,16-Me)
1.26(3H,s,17-Me)
1.80(3H,s,18-Me)
1.68(3H,s,19-Me)
4.29(1H,d,J=8.2Hz,20α -H)
4.18(1H,d,J=8.2Hz,20β -H)
4.71(1H,m,2'-H)
5.61(1H,dd,J=8.9,2.6Hz,3'-H)
6.50(1H,d,J=8.9Hz,3'-NH)
8.12(2H,dd,J=8.5,1.7Hz,2-OBz(ortho)-H)
7.50(2H,dd,J=8.5,8.3Hz,2-OBz(meta)-H)
7.61(1H,dd,J=8.3,1.7Hz,2-OBz(para)-H)
7.41(2H,m,3'-Ph(ortho)-H)
7.40(2H,m,3'-Ph(meta)-H)
7.35(1H,m,3'-Ph(para)-H)
1.80(3H,s,2''-Me)
6.43(1H,q,J=6.9Hz,3''-H)
1.72(3H,d,J=6.9Hz,3''-Me)
¹³C-NMR(67.8MHz,CDCl₃ , δ c):
C-1 79.0(s) C-1' 172.7(s)
C-2 74.9(d) C-2' 73.3(d)
C-3 45.6(d) C-3' 54.8(d)
C-4 81.1(s) 4-OCOCH₃ 170.3(s)
C-5 84.4(d) 4-OCOCH₃ 22.6(q)
C-6 35.8(t) 10-OCOCH₃ 171.2(s)
C-7 72.1(d) 10-OCOCH₃ 20.8(q)
C-8 58.6(s) 2-COC₆H₅ 167.0(s)
C-9 203.6(s) 2-COC₆H₅ (q)129.1(s)
C-10 75.5(d) (o)130.2(d)
C-11 133.1(s) (m)128.7(d)
C-12 142.1(s) (p)133.7(d)
C-13 72.7(d) 3'-C₆H₅ (q)131.2(s)
C-14 35.6(t) (o)126.9(d)
C-15 43.1(s) (m)128.7(d)
C-16 21.8(q) (p)131.9(d)
C-17 26.8(q) C-1'' 169.0(s)
C-18 14.8(q) C-2'' 138.1(s)
C-19 9.5(q) C-3'' 128.2(d)
C-20 76.5(t) 2''-CH₃ 12.4(q)
3''-CH₃ 13.9(q)

## Claims

1. A process for isolating taxol which comprises extracting taxol from Taxus sumatrana(Taxaceae).

2. The process for isolating taxol according to Claim 1, wherein taxol is extracted from the leaves of Taxus sumatrana(Taxaceae).

3. The process for isolating taxol according to Claim 1 or 2, wherein taxol is extracted with an organic solvent.

4. The process for isolating taxol according to Claim 3, wherein the organic solvent is dichloromethane and/or methanol.

5. The process for isolating taxol according to Claim 1, 2, 3 or 4, wherein taxol is purified by partition between water and a water-immiscible organic solvent and subsequent chromatography.
